# EUROPEAN PATENT APPLICATION

(11) **EP 4 279 043 A1**
(43) Date of publication of application: **22.11.2023**
(21) Application number: 22173782.8
(22) Date of filing: 17.05.2022
(51) Int. Cl.: A61F 13/49, A61F 13/511, A61F 13/84

(54) **ABSORBENT ARTICLE WITH PATTERNED ACTIVE MATERIAL**

(71) Applicant: Ontex BV, 9255 Buggenhout (BE); Ontex Group NV, 9320 Erembodegem (BE)
(72) Inventor: Van Sande, Nathalie, 9688 Maarkedal (BE); WEBER, Ainas, 53474 Bad Neuenahr-Ahrweiler (DE)
(74) Representative: Macchetta, Andrea

(57) **Abstract**

An absorbent article comprising: a topsheet; a backsheet; and an absorbent core sandwiched between said topsheet and backsheet; said core comprising one or more channels being positioned inboard of an outer perimeter of said core formed by two opposed lateral edges connecting two opposed transversal ends of said core; wherein said article comprises an active composition that is applied in a pattern to one or more layers forming said article within an application zone being inboard of at least a portion of said outer perimeter and wherein said application zone is positioned inboard and/or outboard of said one or more channels such that it substantially does not overlap said one or more channels, said active composition providing skin care and/or odour control.

## Description

### TECHNICAL FIELD

The disclosure relates to absorbent articles such as disposable absorbent articles, preferably selected from the group consisting of diapers (whether for baby or adults), pants (whether for baby or adults), briefs, pantyliners, slips, sanitary napkins or towels, preferably sanitary napkins for light incontinence.

### BACKGROUND

Articles comprising channelled cores are becoming more and more sought after in the market in view of their improved liquid distribution properties and reduction in amount of absorbent material used. Examples are described in the literature such as EP 3 342 386 A1, EP 3 692 963 A1, EP 3 542 766 A1, EP 3 738 562 A1 and the like. The preferred means of creating such channels is consistently by bonding directly upper and lower layers of an enveloping core wrap sheet in such a way to create channel-forming areas that are substantially free of absorbent material.

A need has been identified to include active materials in one or more layers of the absorbent article in order to provide benefits such as reduced malodour generally associated to exudates such as urine that is typically associated with incontinent subjects.

The inventors have found that depending on the location of application of such active materials certain drawbacks to core integrity and/or liquid distribution properties are attained as will be explained hereunder in more detail. There thus remains a need for an absorbent article that can provide the excellent liquid distribution with in addition added active functionality, such as odour control, whilst retaining the liquid distribution functionality and core integrity in a cost effective manner.

### SUMMARY

In a first aspect, the disclosure relates to an absorbent article comprising: a topsheet; a backsheet; and an absorbent core sandwiched between said topsheet and backsheet; said core one or more channels being positioned inboard of an outer perimeter of said core formed by two opposed lateral edges connecting two opposed transversal ends of said core; wherein said article comprises an active composition that is applied in a pattern to one or more layers forming said article within an application zone being inboard of at least a portion of said outer perimeter (typically wherein said portion comprises at least one, preferably both, said lateral edges) and wherein said application zone is positioned inboard and/or outboard of said one or more channels such that it substantially does not overlap said one or more channels, said active composition providing skin care and/or odour control.

In a second aspect, the disclosure relates to a method for the manufacture of absorbent articles, said method comprising the steps of: providing a first web of material, preferably a liquid permeable nonwoven; depositing absorbent material onto said first web such that one or more areas of said first web material remain substantially void of absorbent material, said one or more areas corresponding to one or more attachment areas; providing a second web of material, preferably a liquid permeable nonwoven, and applying said second web of material over the deposited absorbent material, or folding said first web to enclose the deposited absorbent material therein; joining the first and second webs together, or the folded first web, at the one or more attachment areas to form an absorbent core having one or more channels substantially free of absorbent material substantially flanked by absorbent material; optionally applying an acquisition distribution layer; optionally sandwiching the absorbent core, and preferably the acquisition distribution layer, between a liquid permeable layer and a substantially liquid impermeable layer oppositely disposed, preferably with the acquisition distribution layer being in contact with the liquid permeable layer; wherein an active composition is applied in a pattern to one or more of the first web of material, second web of material, absorbent material, and acquisition distribution layer, within an application zone being inboard of at least a portion of an outer perimeter of the absorbent core and wherein said application zone is positioned inboard and/or outboard of said one or more channels such that it substantially does not overlap said one or more channels, preferably wherein the first and second webs, or the folded first web, are joined at the one or more attachment areas by one or more adhesives and/or mechanical bonds.

### BRIEF DESCRIPTION OF THE FIGURES

**FIG. 1** is a plan view representation of an article according to an embodiment of the present disclosure.
**FIG. 2A-B** are plan view representations of an article according to embodiments of the present disclosure.
**FIG. 3** is a plan view representation of an article according to an embodiment of the present disclosure.
**FIG. 4** is a cross-section view representation of an article of Fig.1-3 according to an embodiment of the present disclosure.
**FIG. 5** is an illustration of an apparatus according to an embodiment of the present disclosure.

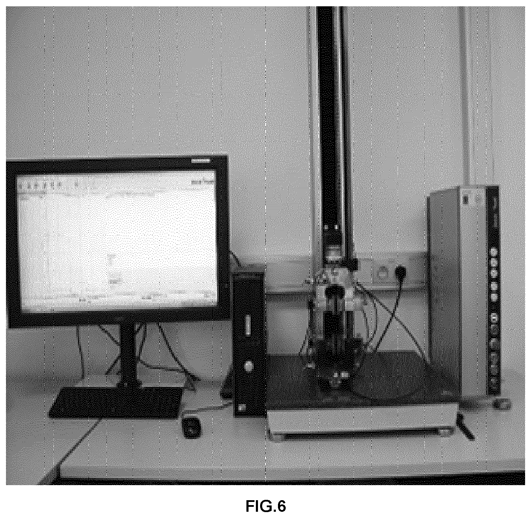

**FIG. 6** is a picture illustrating the Dynamometer Zwick Drawing bench used for test methods herein.

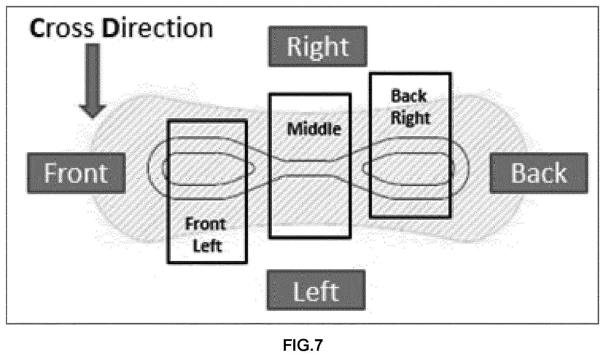

**FIG. 7** is a schematic drawing of articles for test methods described herein.

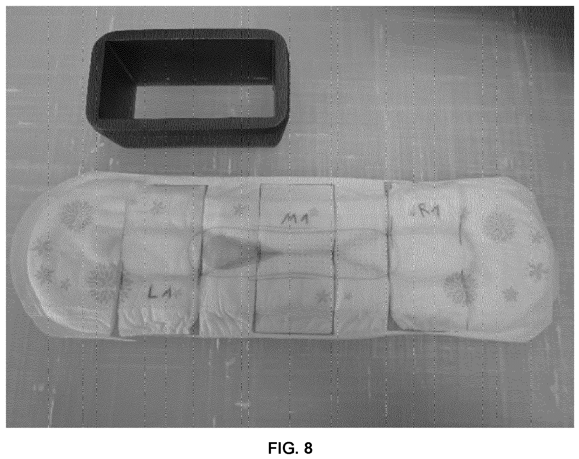

**FIG. 8** is a picture of an absorbent article with left, middle, right positions indicated for test methods herein.

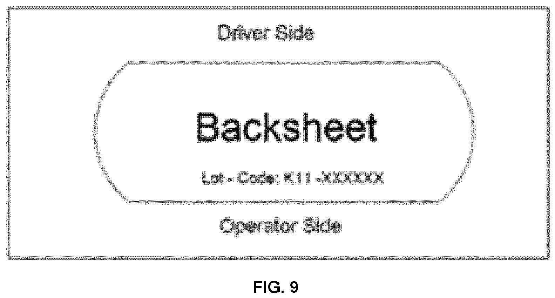

**FIG. 9** is a schematic drawing showing operator side and driver side positions for test methods herein.

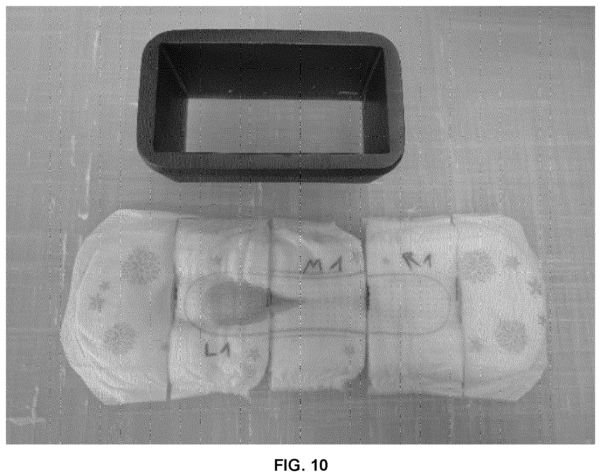

**FIG. 10** is a picture of an absorbent article with left, middle, right cut samples for test methods herein.

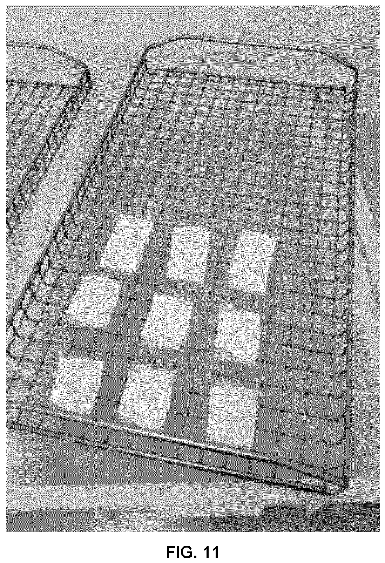

**FIG. 11** is a picture of a drying rack suitable for use in test methods herein.

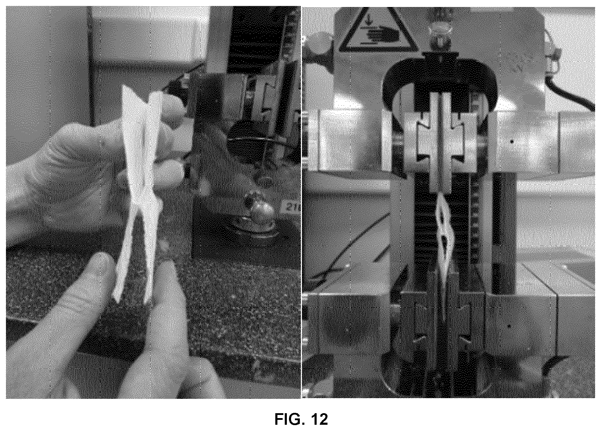

**FIG. 12** is a picture showing the sample mounting for test methods herein.

### DETAILED DESCRIPTION

Unless otherwise defined, all terms used in disclosing characteristics of the disclosure, including technical and scientific terms, have the meaning as commonly understood by one of ordinary skill in the art to which this disclosure belongs. By means of further guidance, term definitions are included to better appreciate the teaching of the present disclosure.

As used herein, the following terms have the following meanings:
"A", "an", and "the" as used herein refers to both singular and plural referents unless the context clearly dictates otherwise. By way of example, "a compartment" refers to one or more than one compartment.

"About" or "substantially" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1% or less of and from the specified value, in so far such variations are appropriate to perform in the disclosed disclosure. However, it is to be understood that the value to which the modifier "about" or "substantially" refers is itself also specifically disclosed.

"Comprise", "comprising", and "comprises" and "comprised of' as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

The expression "% by weight" or "%wt" (weight percent), here and throughout the description unless otherwise defined, refers to the relative weight of the respective component based on the overall weight of the formulation.

The use of the term "layer" can refer, but is not limited, to any type of substrate, such as a woven web, nonwoven web, films, laminates, composites, elastomeric materials, absorbent materials (such as SAP and cellulose fibers/fluff mixtures), or the like. A layer can be liquid and air permeable, permeable to air but impermeable to liquids, impermeable both to air and liquid, or the like. When used in the singular, it can have the dual meaning of a single element or a plurality of elements, such as a laminate or stacked plural sub-layers forming a common layer.

The terms "nonwoven", nonwoven layer" or "nonwoven web" are used interchangeably to mean an engineered fibrous assembly, primarily planar, which has been given a designed level of structural integrity by physical and/or chemical means, excluding weaving, knitting or papermaking (ISO 9092:2019 definition). The directionally or randomly orientated fibers, are bonded by friction, and/or cohesion and/or adhesion. The fibers may be of natural or synthetic origin and may be staple or continuous filaments or be formed in situ. Commercially available fibers have diameters ranging from less than about 0.001 mm to more than about 0.2 mm and they come in several different forms such as short fibers (known as staple, or chopped), continuous single fibers (filaments or monofilaments), untwisted bundles of continuous filaments (tow), and twisted bundles of continuous filaments (yam). Nonwoven webs can be formed by many processes such as meltblowing, spunbonding, solvent spinning, electrospinning, carding and airlaying. The basis weight of nonwoven webs is usually expressed in grams per square meter (g/m2 or gsm).

The term "disposable" refers to absorbent articles and/or inserts that generally are not intended to be laundered or otherwise restored or reused as absorbent articles, i.e., they are intended to be discarded after a single use and, preferably, to be recycled, composted or otherwise disposed of in an environmentally compatible manner.

The term "disposed" is used to mean that an element(s) is formed (joined and positioned) in a particular place or position as a unitary structure with other elements or as a separate element joined to another element.

The terms "interior" and "exterior" refer respectively to the location of an element that is intended to be placed against or toward the body of a wearer when an absorbent article is worn and the location of an element that is intended to be placed against or toward any clothing that is worn over the absorbent article. Synonyms for "interior" and "exterior" include, respectively, "inner" and "outer", as well as "inside" and "outside", or "body-facing" and "garment-facing". Also, when the absorbent article is oriented such that its interior faces upward, e.g., when it is laid out in preparation for setting the wearer on top of it, synonyms include "upper" and "lower" and "top" and "bottom", respectively.

The term "joined" refers to configurations whereby an element is directly secured to another element by attaching the element directly to the other element, and configurations whereby an element is indirectly secured to another element by attaching the element to intermediate member(s) which in turn are attached to the other element.

The term "lateral" or "transverse" refers to a direction running at a 90 degree angle to the longitudinal direction and when combined with the term "substantially" includes directions within ±45° of the lateral direction.

The term "longitudinal" refers to a direction running parallel to the maximum linear dimension of the article and when combined with the term "substantially" includes directions within ±45° of the longitudinal direction.

An "anti-bacterial agent" is defined in the present invention as a compound which is able to either kill bacteria, such as ammonia-generating bacteria which exist in the urogenital region of humans, or to suppress the growth of said bacteria.

Embodiments of the articles according to the disclosure will now be described. It is understood that technical features described in one or more embodiments maybe combined with one or more other embodiments without departing from the intention of the disclosure and without generalization therefrom, especially when such combinations are explicitly or implicitly inferred.

### THE ARTICLE

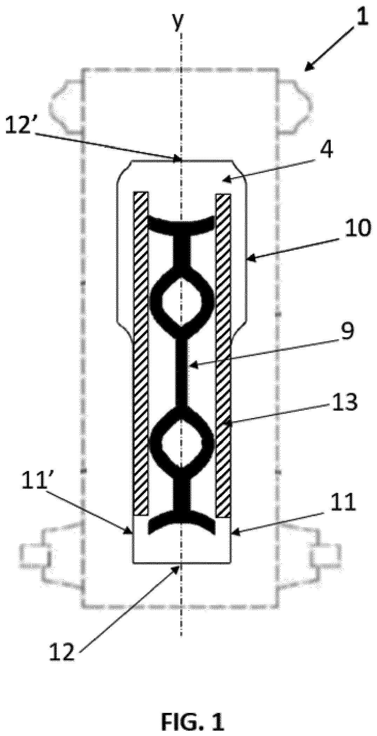

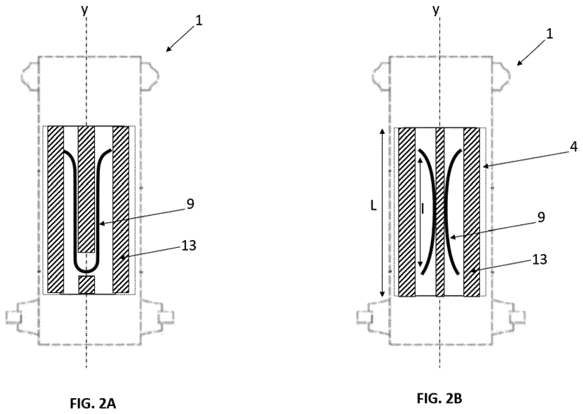

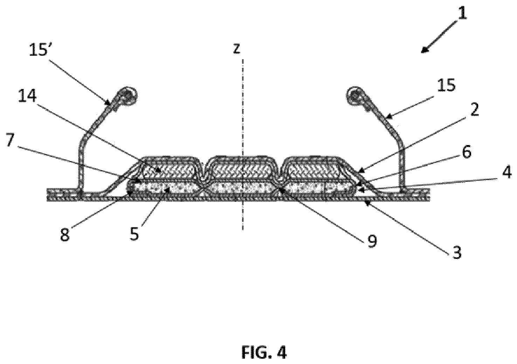

Absorbent articles (1) described herein, and as exemplified in Figs. 1-4, comprise: a topsheet (2); a backsheet (3); and an absorbent core (4) sandwiched between said topsheet (2) and backsheet (3); said core (4) typically comprises absorbent material (5) that is substantially enclosed by a core wrap (6), wherein said core wrap (6) may comprise a top core wrap layer (7) and a bottom core wrap layer (8), and wherein said top and bottom layers are typically joined together along one or more continuous or discontinuous attachment areas forming one or more channels (9), substantially free of absorbent material, and being positioned inboard of an outer perimeter (10) of said core (4) formed by two opposed lateral edges (11, 11') connecting two opposed transversal ends (12, 12') of said core (4); wherein said article (1) comprises an active composition that is applied in a pattern to one or more layers forming said article (1) within an application zone (13) being inboard of at least a portion of said outer perimeter (10), typically wherein said portion comprises at least one, preferably both, said lateral edges (11, 11') or transverse ends (12, 12') or combinations thereof, and wherein said application zone (13) is positioned inboard and/or outboard of said one or more channels (9) such that it substantially does not overlap said one or more channels (9), said active composition providing skin care and/or odour control. "Active composition" as used herein means any composition or formulation that is applied to one or more layers making up the absorbent article and that generally has an effect of improving or reducing odour and/or skin rashes and/or irritations of a wearer. Advantageously the active composition applied in this manner results in the formation of odour "barriers" (in the case of odour control) and/or skin moisturising/soothing at concentrated regions where most needed i.e. between the leg folds (or close thereto) of the wearer that are found to be most prone to sweat and humidity accumulation. At the same time risk of contamination (e.g. by migration of the composition) of the channel regions that may result in weakening of the attachment bond is limited hence promoting core integrity during use and/or wetting.

In a preferred embodiment, the application zone (13) is outboard of said one or more channels (9) and at a distance therefrom. It has been surprisingly found that this leads to improved channel formation as well as odour barriers limiting odour seepage from the sides of the article.

The top and bottom core wrap layers (7, 8) are preferably joined together by one or more adhesives and/or mechanical bonds. Advantageously this permits not only to attain excellent liquid transfer performance along/across the core surface but further also aids to retain core integrity particularly during wetting and swelling of the absorbent material.

Preferably, the pattern comprises, preferably consists of, one or more stripes or swirls. The pattern may comprise, preferably consists of, no more than 3 stripes or swirls, preferably from 1 to 2 stripes or swirls. Advantageously this aids to concentrate the active composition where most needed.

The stripes or swirls may have a maximum width of at least 10mm, preferably from 15 mm to 55 mm, more preferably from 20 mm to 50 mm, even more preferably from greater than 20 mm to 40 mm, most preferably from 22 mm to 35 mm. Without wishing to be bound by theory, widths that are below such sizes lead to less than optimal efficacy in terms of targeted barrier/benefits described herein whilst above results in added cost and increased liquid transfer impairment.

Preferably, the basis weight of active composition is less than 7 gsm (g/m²), preferably from 1 gsm (g/m²) to 6 gsm (g/m²), more preferably from 2 gsm (g/m²) to 5 gsm (g/m²), even more preferably from 3 gsm (g/m²) to 4 gsm (g/m²). Especially when the active composition is an odour control formulation such amounts result in effective odour barriers with reduced contamination risk and overpowering sensation whilst containing cost.

Preferably, the application zone (13) has a first surface area in a planar view and the absorbent core (4) has a second surface area in said planar view, and wherein the first area is less than 15%, preferably less than 10%, even more preferably from 1% to 4%, of the second surface area. Advantageously this aids to limit liquid transfer hindrance to the core and overall liquid absorption performance of the article.

In an embodiment, the one or more channels (9) have a length (I) along a longitudinal axis (y) that is less than a length (L) of the absorbent core (4) extending along said axis (y) such that the one or more channels (9) do not extend to the perimeter (10) of the core (4). Advantageously this aids to limit risk of undesirable leakage from the edges of the core and article.

The channels (9) suitable herein may have different shapes and sizes depending on the needs, some non-limiting examples are illustrated in Figs. 1, 2A and 2B, and 3. It is however understood that any other channel configuration may be suitable.

Preferably, and as mentioned above in respective preferred embodiments, the active composition is applied outboard of said one or more channels (9) so that application zone (13) is located between at least one of said one or more channels (13) and at least a portion of the perimeter (10) of said core (4), and preferably not, typically directly, between two of said one or more of said channels (9). Advantageously this allows the central region of the core to remain free of active composition hence aiding breathability and limiting liquid barrier effects in direct contact with the largest surface of the buttocks and rather be concentrated on the region of the leg folds, the latter particularly beneficial for sanitary napkins for light incontinence.

The active composition may be applied to one or more layers positioned below the topsheet (2) in a thickness-direction being substantially parallel to a z-axis (z) that crosses said topsheet (2), backsheet (3), and absorbent core (4), preferably such that said topsheet (2) is substantially free of said active composition. Advantageously this allows to keep the active composition away from the skin of a wearer until for example weight or in-use.

The active composition may be arranged to migrate from a layer below the topsheet (2) towards a skin/body-facing surface of said topsheet (2). Preferably, the topsheet (2) is apertured to allow said active composition to migrate therethough upon application of pressure. The apertures may have a diameter (e.g. maximum dimension and/or extension in case of non-circular apetures/openings) of from 500 µm to 3800 µm, preferably from 700 µm to 3500 µm, more preferably from 800 µm to 3000 µm, even more preferably from 1000 µm to 2800 µm, more preferably from 1500 µm to 2500 µm. Particularly when the active composition is or comprises a lotion, the apertures of this range allow for the generally highly viscous material to slowly migrate to the surface for gradual application onto the target locations of the subject's skin.

In an embodiment, the article (1) further comprises a liquid distribution layer (14) positioned between the topsheet (2) and the absorbent core (4), and wherein the active composition is applied to said liquid distribution layer (14) and/or absorbent core (4). Preferably the liquid distribution layer is a nonwoven layer selected from the group consisting of a spunbond nonwoven, carded nonwoven, spunlace nonwoven, and combinations thereof. The basis weight of the liquid distribution layer (14) is preferably from 15 gsm (g/m²) to 45 gsm (g/m²), preferably from 18 gsm (g/m²) to 40 gsm (g/m²), even more preferably from 20 gsm (g/m²) to 38 gsm (g/m²). Without wishing to be bound by theory, higher basis weights lead to greater barriers to migration of the active material and moreover result in added cost.

In an embodiment, the active composition is selected from a lotion, an odour control formulation, and combinations thereof.

Preferably, the active composition is selected from an odour control formulation, preferably said odour control formulation comprising: one or more perfumes; one or more oils, an antibacterial agent or at least one alkali metal or alkaline earth metal chloride; an emollient; an organic acid; one or more cyclodextrins; and/or an organic or inorganic zinc salt.

Preferred anti-bacterial agents are capable of yielding when starting at a concentration of about 103 CFU/ml. liquid for each type of bacteria (CFU is colony-forming unit) and at a given concentration of the anti-bacterial agent (e.g. 10-3 g/g dry absorbent core) an amount of bacteria after 12 hours of 105 CFU/ml liquid for each type of bacteria or lower, preferably 104-10 and more preferably 103-102. This can be measured in line with the method "measuring bacteria inhibition in absorbent bodies" as described in WO 00/35505 (page 17, method 3). This method evaluates the capacity to suppress the growth of or to kill at least one bacterial strain selected from the species Escherichia coli, Proteus mirabilis and Enterococcus faecalis.

Anti-bacterial agents for use herein are preferably compounds which are skin-friendly. It needs to be borne in mind that the skin area being in contact with absorbent products such as diaper, panty diaper, sanitary napkin or incontinence device is sensitive and delicate.

Anti-bacterial agents which are approved for the use in food (e.g. as preservatives) are therefore used with preference (for instance those food preservatives being approved at the priority of the present application in any EC member state or the US or Japan).

The anti-bacterial agent may be organic or inorganic. It may for instance be selected from the following organic compounds: isothiazolinones and benzisothiazolinones, oxazolidines, pyridines, optionally chlorinated phenols, bromo compounds, aldehyde and dialdehyde compounds, benzyl alcohols, cresols, p-hydroxybenzoic acids and their esters and salts (parabene compounds), organic acids and their salts, in particular alkali metal and earth alkaline metal salts and organic polyacids and their salts, in particular alkali metal and earth alkaline metal salts. An anti-bacterial agent belongs to the above classes if it displays (comprises) the corresponding structural features. Accordingly, (further) substituted members such as hydroxylated organic acids are also covered by the above classes.

The inorganic anti-bacterial agent may be selected from sulfites, bisulfites, nitrates, nitrites and iodates of alkali metals such as sodium and potassium or earth alkaline metals such as calcium or magnesium.

Preferably, one of the following compounds or a mixture thereof is used as an anti-bacterial agent:
- 1,2-benzisothiazoline-3-one (BIT, Proxel);
- benzoic acid, E 210;
- benzyl alcohol;
- 2-benzyl-4-chlorophenol (Chlorophene);
- 1,3-bis(hydroxymethyl)-5,5-dimethylimidazoline-2,4;
- 5-bromo-5-nitro-1,3-dioxane (Bronidox^{™});
- 2-bromo-2-nitropropane-1,3-diol (BNPD);
- succinic acid dialdehyde;
- dehydroacetic acid (6-methylacetopyranone);
- diazolidinyl urea (Germall II^{™});
- 1,2-dibromo-2,4-dicyanobutane;
- 6,6-dibromo-4,4-dichloro-2,2'-methylenediphenol;
- 3,3'-dibromo-4,4'-hexamethylene dioxydibenzamindine (dibromohexamidine);
- 2,4-dichlorobenzyl alcohol;
- 5,5'-dichloro-2,2'-dihydroxydiphenylmethane (Dichlorophen);
- 4,4-dimethyl-1,3-oxazolidine;
- phenols, e.g. o-phenylphenol;
- cresols, e.g. o-, m- or p-cresol, 4-isopropyl-m-cresol, p-chloro-m-cresol;
- 2-phenoxyethanol (ethylene glycol monophenyl ether);
- 1-phenoxypropane-2-ol;
- o-phenylphenol and salts thereof;
- phenyl mercury silver salts including borates;
- formaldehyde;
- fumaric acid, E 297;
- glutaraldehyde;
- glyoxal;
- hexetidine;
- hexamethylenetetramine, E 239;
- p-hydroxybenzoic acid (4-hydroxybenzoic acid);
- p-hydroxybenzoic acid-benzylester (benzyl parabene);
- p-hydroxybenzoic acid-n-butylester (butyl parabene);
- p-hydroxybenzoic acid-ethylester, E 214 (ethyl parabene);
- p-hydroxybenzoic acid-ethylester sodium salt, E 215 (ethylparabene sodium salt);
- p-hydroxybenzoic acid-n-heptylester (heptyl parabene);
- p-hydroxybenzoic acid-methylester, E 218 (methyl parabene);
- p-hydroxybenzoic acid-methylester sodium salt, E 219 (methyl parabene sodium salt);
- p-hydroxybenzoic acid-n-propylester, E 216 (propyl parabene);
- p-hydroxybenzoic acid-n-propylester sodium salt, E 217 (propyl parabene sodium salt);
- 1-hydroxy-4-methyl-6(2,4,4-trimethylpentyl)-2-pyridone; imidazolidinyl urea;
- calcium acetate, E 263;
- calcium bisulfite, E 227;
- calcium propionate, E 282;
- calcium sulfite, E 226;
- potassium disulfite, E 224 (potassiumpyrosulfite);
- potassium nitrate, E 252;
- potassium propionate, E 283;
- potassium sorbate, E 202;
- 2-chloracetamide;
- N-(3-chloroallyl)-hexaminiumchloride (Quaternium 15);
- 1-(4-chlorphenoxy)-1-(imidazol-1-yl)-3,3-dimethylbutan-2-one;
- Chlorhexidine;
- p-chloro-m-xylenole;
- 5-chloro-2-methyl-4-isothiazoline-3-one;
- 4-chloro-3,5-xylenole;
- metenamine-3-chloroallylchloride;
- n,n'-methylenebis(5-methyl-oxazolidine) (Grotan OD^{™});
- 2,2'-methylene-bis-(3,4,6-trichlorophenol) (Hexachlorophene);
- lactic acid E 270;
- myristic acid;
- natamycin, E 235 (Pimaricin);
- sodium acetate, E 262;
- sodium benzoate, E 211;
- sodium diacetate, E 262;
- sodium forminate, E 237;
- sodium nitrate, E 251;
- sodium nitrite, E 250;
- sodium propionate, E 281;
- sodium-2-pyridinethiol-1-oxide (Omadin^{™} and PyrionNa^{™});
- sodium sorbate, E 201;
- sodium sulfite, E 221;
- sodium disulfite, E 223 (sodium pyrosulfite);
- sodium iodate;
- sodium hydrogensulfite, E 222 (sodium bisulfite);
- Nisin, E 234;
- 2-n-octyl-4-isothiazoline-3-one (Kathon 893^{™} and Skane M-8^{™});
- Paraformaldehyde;
- poly(1-hexamethylene biguanide hydrochloride);
- propionic acid, E 280;
- salicylic acid (2-hydroxybenzoic acid);
- sorbic acid, E 200;
- inorganic sulfites;
- sulfur dioxide (aq.), E 220;
- 2,2'-thio-bis-(4,6-dichlorophenol) (Bithionol);
- Thiomersal (ethyl mercury thiosalicylate);
- 1,3,5-triazine-1,3,5-(2H, 4H, 6H)-triethanol;
- trichlocarban (3,4,4-trichlorocarbanilide);
- 2,4,4'-trichloro-2'-hydroxydiphenylether (Irgasan DP300^{™} and Triclosan^{™});
- 3,4,4-trimethyl-1,3-oxazolidine (Bioban CS1135^{™} and Oxaben A^{™});
- undecene acid;
- inorganic hydrogensulfites;
- zinc-bis-(2-pyridinethiol-1-oxide) (Zink-Omadin^{™});
- malic acid, E 296 (hydroxy succinic acid);
- acetic acid, E 260;
- morpholine derivatives, e.g. 4-(nitrobutyl)-morpholine and
- 4,4'-(2-ethyl-2-nitro-trimethylene)-dimorpholine (Bioban P 1487^{™} or Bioban CS1248^{™});
- oxazolidines;
- pyridine derivatives;
- Kathon CG (mixture of 5-chloro-2-methyl-4-isothiazoline-3-one and 2-methyl-4-isothiazoline-3-one); and
- 1,1,1-trichloro-2-methyl-2-propanol (chlorobutanol).

The anti-bacterial agent is preferably selected from organic acids and polyacids (e.g. diacids or triacids), and their salts. The organic (poly)acid may be substituted by one, two or more hydroxy groups. The organic (poly)acid may be an unsaturated (e.g. mono- or diunsaturated) or saturated, linear or branched aliphatic carboxylic acid preferably having from 2 to 18 carbon atoms, more preferably 3 to 8 carbon atoms. Examples thereof were already mentioned in the above list. The organic acid may also represent an aromatic (poly)acid, preferably a phenyl(poly)carboxy acid, having preferably from 7 to 18 carbon atoms, in particular 7 to 10 carbon atoms as in benzoic acid, p-hydroxybenzoic acid or salicylic acid. The salt is preferably an alkali metal (e.g. K or Na) or earth alkaline metal salt (e.g. Ca or Mg).

More preferably benzoic acid, sorbic acid, tartaric acid or citric acid, most preferably benzoic acid or citric acid, are used as the anti-bacterial agent herein.

The alkali metal chloride may be potassium chloride (KCI) or sodium chloride (NaCI) and is preferably NaCl. The alkaline earth metal chloride may be magnesium chloride (MgCI2) or calcium chloride (CaCI2). The alkali metal chloride or alkaline earth metal chloride, when present, is typically present in an amount of at least 0.01, preferably at least 0.05, more preferably at least 0.1 g per g dry absorbent core.

There are no specific restrictions regarding the organic zinc salt to be used in combination with the component (i). In accordance with the present invention, zinc salts of organic carboxylic acids having 2 to 30 carbon atoms, in particular 12 to 24 carbon atoms are preferably used. The carboxylic acid group may be attached to aliphatic, aliphatic-aromatic, aromatic-aliphatic, alicyclic, or aromatic residues, wherein the aliphatic chain or the alicyclic ring(s) may be unsaturated and are optionally substituted, for instance by hydroxy or C1 to C4 alkyl. These salts include zinc acetate, zinc lactate, zinc ricinoleate and zinc abietate. More preferably, the zinc salt is the zinc salt of an unsaturated hydroxylated fatty acid having 8 to 18 carbon atoms. Although there is no specific restriction regarding the number of unsaturated double bonds or hydroxy groups, those fatty acids having one or two unsaturated double bonds and one or two hydroxyl groups seem to be preferred. The most preferred embodiment is zinc ricinoleate. The organic zinc salt may also be activated by the presence of amino acids as in TEGO^{®} Sorb available from Degussa. Further, the organic zinc salt to be used in the present invention may also be capable of removing malodourous substances chemically based on amines, e.g., nicotine in cigarette smoke, thiocompounds, e.g., allicin in garlic and onions, and acids, e.g., isovaleric acid in human sweat, and butyric acid. For instance, zinc ricinoleate which is, e.g., marketed by Degussa under the tradename TEGO^{®} Sorb has the described additional odour removing effect apart from removing ammonia.

In an embodiment, the anti-bacterial agent is selected from the group consisting of benzoic acid, sorbic acid, tartaric acid and citric acid, or a mixture thereof, and the zinc salt is an organic zinc salt preferably consisting of zinc ricinoleate or an inorganic zinc salt preferably consisting of zinc oxide.

As regards the amount of anti-bacterial agent or at least one alkali metal or alkaline earth metal chloride and zinc salt to be used herein, there are no specific restrictions. In the present specification, these amounts are expressed in relation to the weight (in g) of the dry absorbent core. Herein the term "dry" used in relation to the absorbent core is to be understood such that no water has been added to the absorbent core and that the only water present in the absorbent core is the unavoidable residual water from manufacturing. For the purpose of the present invention, an absorbent core is preferably regarded as "dry" after a circular test core having a thickness of 5 to 6 mm, a diameter of 5 cm and which has been compressed to a bulk of about 8-10 cm3/g has been kept for at least one week at ambient temperature (e.g. 20° C.) and a specified relative humidity, for example 50% RH.

While there are no specific restrictions as to the amount of anti-bacterial agent to be used herein, as long as the objects set forth are not compromised, the amount of anti-bacterial agent is preferably at least 1×10-4 g, more preferably at least 5×10-4 g, most preferably at least 1×10-3 g per g of dry absorbent core. However, there are cases where the antibacterial agent can be used in amounts as low as 5 to 10 ppm of antibacterial agent(s) (by weight) in terms of the dry absorbent core. Such a case is Kathon^{®} CG, which is a mixture of two compounds, namely 5-chloro-2-methyl-4-isothiazoline-3-one and 2-methyl-4-isothiazoline-3-one, Beyond a certain amount of anti-bacterial agent (for instance 0.01 g or 0.1 g per g dry absorbent core), it may no longer be economical to add further anti-bacterial agent.

In an embodiment, the amount of alkali metal chloride or alkaline earth metal chloride, such as sodium chloride is at least 0.01 g, preferably at least 0.05 g, for instance at least 0.1 g per g dry absorbent core. There is no specific upper limit for the (earth) alkali metal chloride content, although it may no longer be beneficial to increase the amount beyond values such as 0.5 g per g absorbent core or 1 g per g absorbent core.

In an embodiment, the weight ratio of the anti-bacterial agent, alkali metal chloride or alkaline earth metal chloride, and the zinc salt is also not specifically limited, but it is preferably 15/1 to 1/5, more preferably 5/1 to 1/2, for instance 3/1 to 1/1.

In a preferred embodiment, the emollients for use herein are selected from the group consisting of stearyl alcohol, petrolatum, and mixtures thereof.

Preferably, the absorbent material (5) comprises superabsorbent polymer particles and/or cellulose fibers.

The article (1) herein may be disposable and selected from the group consisting of diapers, pants, briefs, pantyliners, slips, sanitary napkins and towels, and combinations thereof, preferably sanitary napkins for light incontinence.

The article (1) herein may further comprise oppositely disposed lateral cuffs (15, 15') that generally act as longitudinal barriers to liquid leakage. The cuffs typically comprise a hydrophobic nonwoven layer and one or more elastics proximal to an apex thereof to form stand-up gathers. For clarity of the drawings, only one or two elastic strands are shown for each cuff, but typically each cuff may comprise from 1 to 4 elastic strands. The absorbent article may also comprise other typical components such as a back elastic waist feature, a front elastic waist feature, transverse barrier cuffs, a wetness indicator that changes colour when contacted with urine, etc.

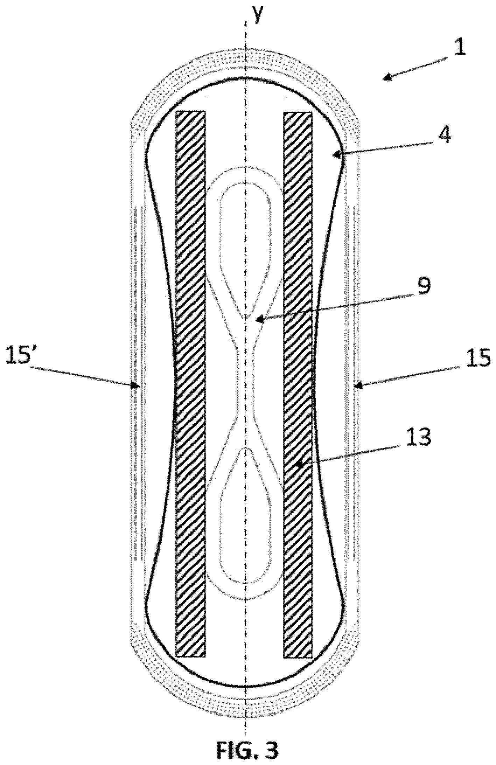

In a highly preferred embodiment, as illustrated in Fig.3, the absorbent article is a sanitary napkin preferably for use in light incontinence (or by continent women). Preferably, the backsheet (3) is substantially liquid impermeable and comprises one or more adhesive stripes on a garment-facing side thereof such to provide a means of attachment to a wearer's undergarment such as underwear or slips. The one or more adhesive stripes may be covered by a paper, preferably siliconized, release strip to maintain the adhesive properties until intended use.

### THE PROCESS

A method for the manufacture of absorbent articles herein comprises the steps of: providing a first web of material, preferably a liquid permeable nonwoven; depositing absorbent material onto said first web such that one or more areas of said first web material remain substantially void of absorbent material, said one or more areas corresponding to one or more attachment areas; providing a second web of material, preferably a liquid permeable nonwoven, and applying said second web of material over the deposited absorbent material, or folding said first web to enclose the deposited absorbent material therein; joining the first and second webs together, or the folded first web, at the one or more attachment areas to form an absorbent core having one or more channels substantially free of absorbent material substantially flanked by absorbent material; optionally applying an acquisition distribution layer; optionally sandwiching the absorbent core, and preferably the acquisition distribution layer, between a liquid permeable layer and a substantially liquid impermeable layer oppositely disposed, preferably with the acquisition distribution layer being in contact with the liquid permeable layer; wherein an active composition is applied, preferably by spraying, in a pattern to one or more of the first web of material, second web of material, absorbent material, and acquisition distribution layer, within an application zone being inboard of at least a portion of an outer perimeter of the absorbent core and wherein said application zone is positioned inboard and/or outboard of said one or more channels such that it substantially does not overlap said one or more channels, preferably wherein the first and second webs, or the folded first web, are joined at the one or more attachment areas by one or more adhesives and/or mechanical bonds.

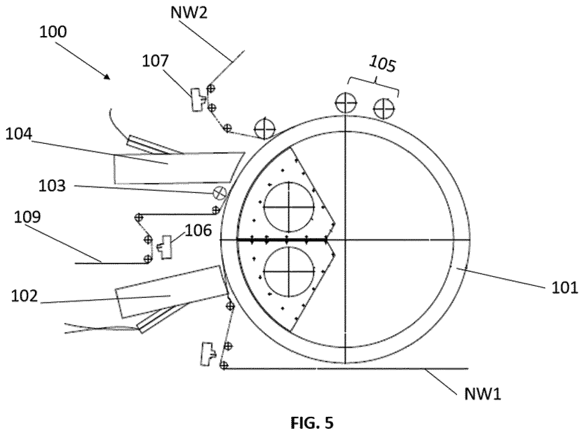

In an embodiment, exemplified in Fig. 5, the method for making an absorbent article comprising the steps of:
i. providing a pocket comprising a single porous cavity, wherein said cavity is in fluid communication with an under-pressure source;
ii. providing a first nonwoven web (NW1) in the form of a bottom layer of a core wrap;
iii. depositing said bottom layer onto said pocket;
iv. depositing a first absorbent material (typically via an airstream, e.g. blowing), comprising cellulose fibers and/or superabsorbent polymer particles, over at least a portion of a surface of said bottom layer;
v. depositing an intermediate layer (109) over the first absorbent material such that said first absorbent material is sandwiched between said intermediate layer (109) and bottom layer;
vi. joining said intermediate layer (109) to said bottom layer at one or more first distinct positions;
vii. depositing a second absorbent material (typically via an airstream, e.g. blowing), comprising cellulose fibers and/or superabsorbent polymer particles, over at least a portion of a said intermediate layer (109);
viii. depositing a second nonwoven web (NW2) in the form of a top layer of a core wrap over the second absorbent material such that said second absorbent material is sandwiched between said intermediate layer (109) and top layer;
ix. joining said intermediate layer (109) to said top layer and/or bottom layer at one or more second distinct positions, to form an absorbent core;
x. optionally joining an acquisition distribution layer to the body facing surface of said top layer, and preferably laminating the absorbent core and the acquisition distribution layer between a liquid pervious topsheet and a liquid impervious backsheet;
   wherein the absorbent core (4) comprises no more than two compartments or clusters of absorbent material corresponding to said single cavity, and typically at most a top compartment or cluster between the top layer and the intermediate layer (109); and a bottom compartment or cluster between the bottom layer and the intermediate layer (109).

In an embodiment, the first and second distinct positions are the same or different, and wherein said first and/or second distinct positions comprise one or more bonding points positioned inboard of the perimeter of the absorbent core (4), and preferably wherein said bonding points have an aspect ratio of less than 3, preferably of from 1 to 2, and more preferably having a shape selected from the group consisting of circular, elliptical, line-form, star-shaped, polygonal, and combinations thereof, and preferably wherein said bonding points comprise mechanical bonds.

In an embodiment, the method further comprises the step of locally removing the absorbent material applied on said bottom and/or intermediate layers, preferably by a mechanical removal means preferably comprising one or more roller brush or air blower.

In an embodiment, the method further comprises the step of applying a first adhesive pattern on a surface of the intermediate layer facing the first absorbent material; and applying an second adhesive pattern on a surface of the top layer facing the second absorbent material, preferably wherein the first and second patterns are substantially the same or different.

Preferably, the joining step(s) (at least the joining of the intermediate layer (9) to the bottom layer (8)) comprises the step of applying a pressure and/or adhering force to join the first, second, and/or third sheet materials respectively, substantially concurrently with a suction force within the at least one suction zone typically such to combine a downward push with a substantially simultaneous downward pull wherein downward is the direction from the position at which pressure is applied to or towards the supporting member. This is typically achieved by ensuring that the attachment unit(s) herein is/are disposed such to superpose a vacuum region (V) within the support unit (typically in the form of a rotating drum). Advantageously this allows to attain good and strong adhesion without having to apply excessive pressures with the use of e.g. pressure rollers (or embossing rollers) that may inadvertently damage sections of the absorbent core.

In an embodiment, the apparatus (100) used to make absorbent articles according to embodiments herein comprises:
a supporting member (101) for supporting a first sheet material (being the bottom layer (8) along a surface thereof, typically said supporting member (101) comprising a plurality of said pockets generally disposed along a circumference thereof, wherein the surface of said supporting member (101) is provided with at least one suction zone and at least one non-suction zone;
a first application unit (102) configured for applying a first absorbent material (typically by depositing absorbent material via an airstream, e.g. blowing) on said first sheet material on the supporting member (101); said first absorbent material being applied such that said first absorbent material is located on a portion of the first sheet material corresponding to the at least one suction zone, and wherein substantially no absorbent material is present on other one or more portions of the first sheet material corresponding to the at least one non-suction zone on at least one first attachment portion (herein also zone or area);
a first sheet feed unit configured for applying a second sheet material (being the intermediate layer (109)) on top of the first absorbent material on the first sheet material; optionally a first attachment unit (103) configured for attaching said first sheet material to said second sheet material at least in the at least one first attachment portion;
a second application unit (104) configured for applying a second absorbent material (typically by depositing absorbent material via an airstream, e.g. blowing) on said second sheet material and/or first absorbent material; said second absorbent material being applied such that said second absorbent material is located on a portion of the second sheet material, and wherein substantially no absorbent material is present on other one or more portions of the second sheet material on at least one second attachment portion;
a second sheet feed unit configured for applying a third sheet material (being the top layer) on top of the second absorbent material on the second sheet material;
a second attachment unit (105) configured for attaching said second sheet material to said third sheet material at least in the at least one second attachment portion;
   wherein the first attachment portion and the second attachment portion are substantially congruent or substantially complementary (typically when viewed in a planar view of the absorbent core).

In a preferred embodiment, at least the first application unit (102) (but preferably all the application units described herein) comprises a blowing means (such as an air stream source) to direct the absorbent material onto the bottom layer/first sheet material and/or the intermediate layer/second sheet material, and is preferably a non-contact application unit in that it is free of a rotatable laying-out roller comprising a plurality of pockets for applying a spread of absorbent material (also conventionally known or referred to as absorbent material printing). Especially when the absorbent material comprises cellulose fibers it is desirable to apply such absorbent material via a non-contact application in order to achieve good mixing and spacing apart of the superabsorbent polymer particles between cellulose fibers and limit agglomeration of said particles, this allowing to achieve cores with better liquid distribution properties along and across its surface.

In an embodiment, the supporting member (101) is a rotating drum and the at least one non-suction zone comprises at least one elongate zone extending in a circumferential direction of the rotating drum, preferably a plurality of said non-suction zones are comprised in said pocket. Preferably, the at least one non-suction zone is formed by at least one element being substantially planar with an outer surface of the rotating drum; and wherein the at least one suction zone is formed by at least one cavity comprising a substantially porous base that is positioned at a first radial distance from the center of said rotating drum being less than a second radial distance of said outer surface from said center.

In an embodiment, the apparatus further comprises a removing unit comprising a mechanical removal means configured for locally removing the absorbent material applied on said first and/or second sheet material above the at least one non-suction zone. Typically, wherein the mechanical removal means comprises one or more roller brush or air blower.

Preferably, the first sheet feed unit is positioned between the first application unit and the second application unit, and typically upstream of the first attachment unit (103) along a machine direction (MD). Advantageously this allows to ensure reduced risk of contamination between layers.

In an embodiment, the apparatus further comprises a first adhesive unit (106) arranged to apply an adhesive pattern on a surface of the second sheet material facing the first absorbent material; and a second adhesive unit (107) arranged to apply an adhesive pattern on a surface of the third sheet material facing the second absorbent material; said first and second adhesive units being positioned upstream of said first and second attachment units (103, 105) respectively. The apparatus may further comprise a further adhesive unit arranged to apply an adhesive pattern on a surface of the first sheet material facing the first absorbent material.

In an embodiment, the first and second attachment units comprise a pressure means, such as a pressure roller (typically having a substantially smooth contact surface generally such that it is free of protrusions pressing into the channel forming areas so as to limit the need for process registration), arranged to provide an adhering force to join the first, second, and third sheet materials respectively; and preferably wherein the first attachment unit comprises a single pressure means, and the second attachment unit comprises a plurality, preferably from 2 to 5, of pressure means.

In an embodiment, the first attachment unit is positioned between the first application unit and the second application unit, and downstream of the first sheet feed unit, typically along a machine direction (MD). Advantageously this allows to ensure optimal adhesion and reduced risk of contamination between layers.

### TEST METHODS

### Channel integrity:

The following procedure is carried out in order to determine the bonding strength of top to bottom core wrap layers in channel(s) herein.

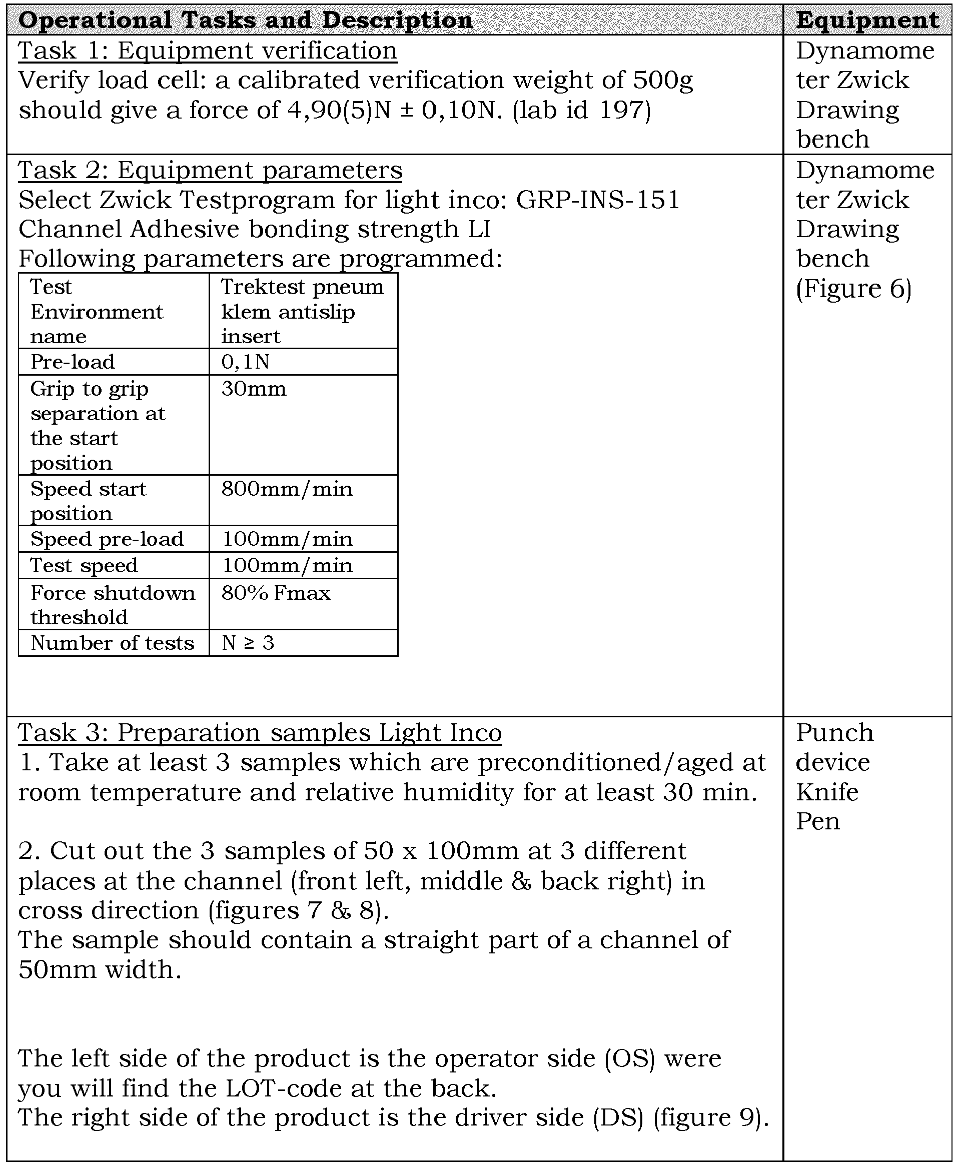

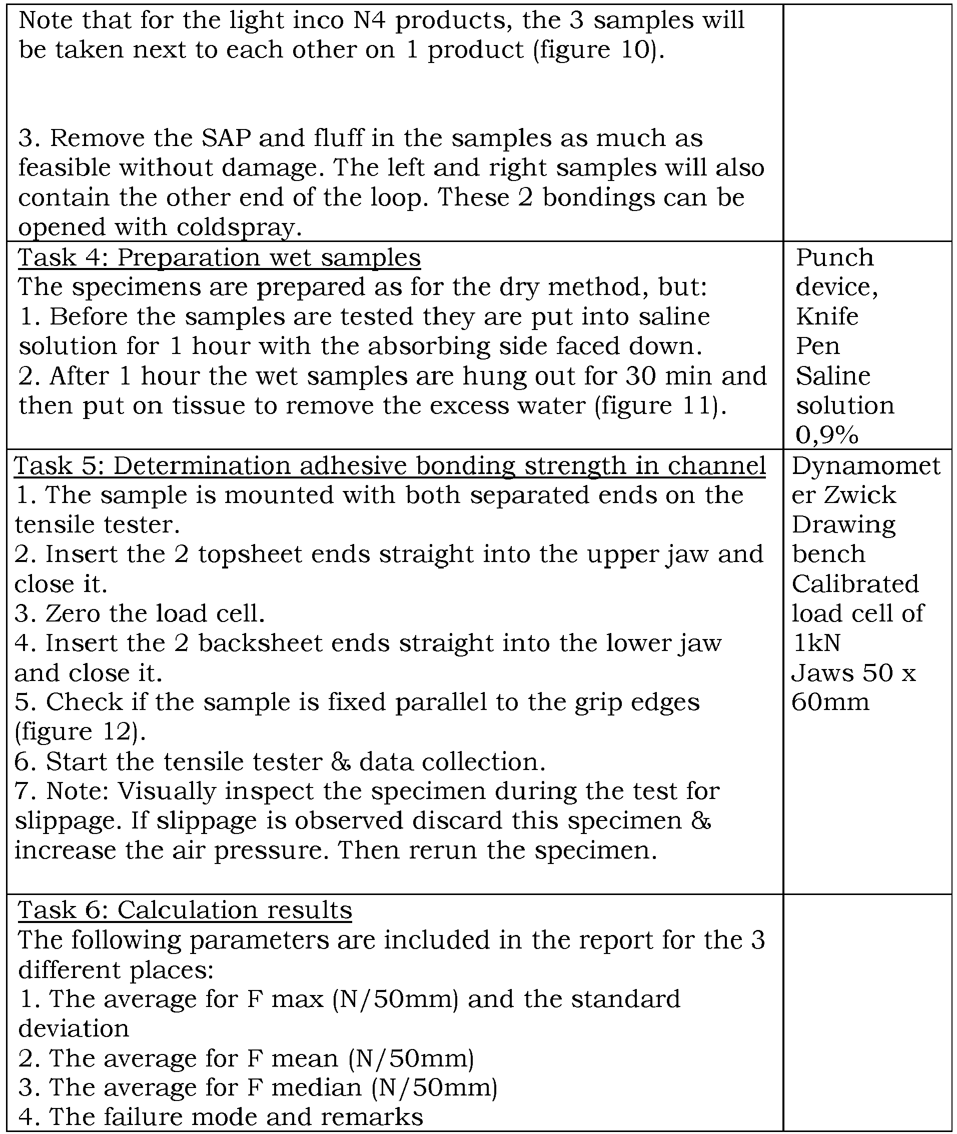

### EXAMPLES

A total of 28 sanitary napkins for light incontinence are made. Each napkin resembles the schematic embodiment of Fig.3 including channel(s) as described and shown with the same construction and adhesive type and amount for joining top and bottom core wrap layers to form the channel(s), and contains the same amount in gsm of an identical odour control (OC) formulation that is applied as follows: **REFERENCE 1** [7 samples with odour control formulation applied to the top core wrap over substantially the full surface including the channel(s)]; **SAMPLE A** [7 samples with odour control formulation applied to the top core wrap inboard of the channel(s)]; **SAMPLE B** [7 samples with odour control formulation applied to the top core wrap ouboard of the channel(s)]; **CONTROL** [7 samples without odour control formulation].

Each sample is tested according to the channel integrity test method herein but for 7 samples of each set described above, and an average taken for the total of the 7 sample sets at all 3 locations described. The results are reported in table 1 below.

**Table 1 - Channel bonding force results**

| **EXAMPLES** | **Average Fmax (N/50mm)** |
|---|---|
| CONTROL: No OC | 14.0 |
| REFERENCE 1: OC over channel(s) | 2.8 |
| SAMPLE A: OC inboard of channel(s) | 11.2 |
| SAMPLE B: OC outboard of channel(s) | 14.0 |

As can be seen Sample A and B achieve almost equivalent bonding strength as the Control sample. With Sample B even showing exceptional performance compared to sample A itself.

It is supposed that the present invention is not restricted to any form of realization described previously and that some modifications can be added to the presented embodiments without reappraisal of the appended claims.

## Claims

1. An absorbent article (1) comprising:
a topsheet (2);
a backsheet (3); and
an absorbent core (4) sandwiched between said topsheet (2) and backsheet (3);
said core (4) comprising one or more channels (9) being positioned inboard of an outer perimeter (10) of said core (4) formed by two opposed lateral edges (11, 11') connecting two opposed transversal ends (12, 12') of said core (4); **characterized in that** said article (1) comprises an active composition that is applied in a pattern to one or more layers forming said article (1) within an application zone (13) being inboard of at least a portion of said outer perimeter (10) and wherein said application zone (13) is positioned inboard and/or outboard of said one or more channels (9) such that it substantially does not overlap said one or more channels (9), said active composition being suitable for providing skin care and/or odour control.

2. An absorbent article (1) according to Claim 1 wherein the core (4) comprises absorbent material (5) that is substantially enclosed by a core wrap (6), wherein said core wrap (6) comprises a top core wrap layer (7) and a bottom core wrap layer (8), and wherein said top and bottom layers are joined together along one or more continuous or discontinuous attachment areas forming one or more channels (9) substantially free of absorbent material.

3. An absorbent article (1) according to any of the preceding Claims wherein the one or more channels (9) have a length (I) along a longitudinal axis (y) that is less than a length (L) of the absorbent core (4) extending along said axis (y) such that the one or more channels (9) do not extend to the perimeter (10) of the core (4).

4. An absorbent article (1) according to any of the preceding Claims wherein the active composition is applied outboard of said one or more channels (9) so that application zone (13) is located between at least one of said one or more channels (13) and at least a portion of the perimeter (10) of said core (4), and preferably not between two of said one or more of said channels (9).

5. An absorbent article (1) according to any of the preceding Claims wherein the active composition is applied to one or more layers positioned below the topsheet (2) in a thickness-direction being substantially parallel to a z-axis (z) that crosses said topsheet (2), backsheet (3), and absorbent core (4), preferably such that said topsheet (2) is substantially free of said active composition.

6. An absorbent article (1) according to any of the preceding Claims wherein said article (1) comprises a liquid distribution layer (14) positioned between the topsheet (2) and the absorbent core (4), and wherein the active composition is applied to said liquid distribution layer (14) and/or absorbent core (4).

7. An absorbent article (1) according to any of the preceding Claims wherein the active composition is selected from a lotion, an odour control formulation, and combinations thereof.

8. An absorbent article (1) according to any of the preceding Claims wherein the active composition is selected from an odour control formulation, preferably said odour control formulation comprising: one or more perfumes; one or more oils, an anti-bacterial agent or at least one alkali metal or alkaline earth metal chloride; an emollient; an organic acid; one or more cyclodextrins; and/or an organic or inorganic zinc salt.

9. An absorbent article (1) according to any of the preceding Claims wherein the absorbent material (5) comprises superabsorbent polymer particles and/or cellulose fibers.

10. An absorbent article (1) according to any of the preceding Claims wherein said article (1) is disposable and selected from the group consisting of diapers, pants, briefs, pantyliners, slips, sanitary napkins and towels, and combinations thereof, preferably sanitary napkins for light incontinence.

11. An absorbent article (1) according to any of the preceding Claims wherein the pattern comprises, preferably consists of, one or more stripes or swirls.

12. An absorbent article (1) according to Claim 11 wherein the pattern comprises, preferably consists of, no more than 3 stripes or swirls, preferably from 1 to 2 stripes or swirls.

13. An absorbent article (1) according to any one of Claims 11 to 12 wherein the stripes or swirls have a maximum width of at least 10mm, preferably from 15 mm to 55 mm, more preferably from 20 mm to 50 mm, even more preferably from greater than 20 mm to 40 mm, most preferably from 22 mm to 35 mm.

14. An absorbent article (1) according to any of the preceding Claims wherein the basis weight of active composition is less than 7 gsm (g/m²), preferably from 1 gsm (g/m²) to 6 gsm (g/m²), more preferably from 2 gsm (g/m²) to 5 gsm (g/m²), even more preferably from 3 gsm (g/m²) to 4 gsm (g/m²).

15. An absorbent article (1) according to any of the preceding Claims wherein the application zone (13) has a first surface area in a planar view and the absorbent core (4) has a second surface area in said planar view, and wherein the first area is less than 15%, preferably less than 10%, even more preferably from 1% to 4%, of the second surface area.

16. A method for the manufacture of absorbent articles, said method comprising the steps of:
providing a first web of material, preferably a liquid permeable nonwoven;
depositing absorbent material onto said first web such that one or more areas of said first web material remain substantially void of absorbent material, said one or more areas corresponding to one or more attachment areas;
providing a second web of material, preferably a liquid permeable nonwoven, and applying said second web of material over the deposited absorbent material, or folding said first web to enclose the deposited absorbent material therein;
joining the first and second webs together, or the folded first web, at the one or more attachment areas to form an absorbent core having one or more channels substantially free of absorbent material substantially flanked by absorbent material;
optionally applying an acquisition distribution layer;
optionally sandwiching the absorbent core, and preferably the acquisition distribution layer, between a liquid permeable layer and a substantially liquid impermeable layer oppositely disposed, preferably with the acquisition distribution layer being in contact with the liquid permeable layer;
**characterized in that** an active composition is applied, preferably by spraying, in a pattern to one or more of the first web of material, second web of material, absorbent material, and acquisition distribution layer, within an application zone being inboard of at least a portion of an outer perimeter of the absorbent core and wherein said application zone is positioned inboard and/or outboard of said one or more channels such that it substantially does not overlap said one or more channels, preferably wherein the first and second webs, or the folded first web, are joined at the one or more attachment areas by one or more adhesives and/or mechanical bonds.

17. An absorbent article (1) according to any of Claims 2 to 15 wherein the top and bottom core wrap layers (7, 8) are joined together by one or more adhesives and/or mechanical bonds.
